# FASCICULE DE BREVET EUROPEEN

(11) **EP 2 300 407 B1**
(45) Date de publication et mention de la délivrance du brevet: **07.03.2018**
(21) Numéro de dépôt: 09750043.3
(22) Date de dépôt: 05.05.2009
(51) Int. Cl.: C07C 50/22, C07C 69/28, C09B 13/04, C07C 49/747, C07C 49/753, C09B 5/62

(54) **NOUVELLES QUINONES, LEURS SYNTHESES ET UTILISATIONS.**
NEUE CHINONE, SYNTHESEN UND ANWENDUNGEN DAVON
NOVEL QUINONES, SYNTHESES AND USES THEREOF

(30) Priorité: 09.05.2008 FR 0853058
(43) Date de publication de la demande: 30.03.2011
(73) Titulaire: Essilor International, 94220 Charenton-le-Pont (FR); Centre National de la Recherche Scientifique (C.N.R.S.), 75794 Paris Cedex 16 (FR)
(72) Inventeur: BUFFET, Noémie, F-94220 Charenton-le-Pont (FR); BOCK, Harald, F-33600 Pessac (FR)
(74) Mandataire: Cabinet Plasseraud
(86) Numéro de dépôt international: PCT/FR2009/050830
(87) Numéro de publication internationale: WO 2009/141562

(56) Documents cités:
- WO-A1-2006/111511
- WO-A2-2004/074383
- FR-A1- 2 857 858
- JP-A- 2 287 457
- US-A1- 2006 054 987
- PORAI-KOSHITS, CHIZHEVSKAYA: THE JOURNAL OF GENERAL CHEMISTRY OF THE U.S.S.R., vol. 26, no. 7, 1956, pages 2175-2181, XP009110533
- IDEN R. ETAL: "BMFT-FB-T 84-164 : Fluoroszentzfarbstoffe für Sonnenkollektoren." août 1984 (1984-08), FORSCHUNGSBERICHT- BUNDESMINISTERIUM FÜR FORSCHUNG UND TECHNOLOGIE , XP002549554 pages 26,77,87,96;composé 20
- LEE SANG KWON ET AL: "Electrochemistry, Spectroscopy and Electrogenerated Chemiluminescence of Perylene, Terrylene, and Quaterrylene Diimides in Aprotic Solution" JOURNAL OF THE AMERICAN CHEMICAL SOCIETY, AMERICAN CHEMICAL SOCIETY, WASHINGTON, DC. US, vol. 121, 1 janvier 1999 (1999-01-01), pages 3513-3520, XP002252150 ISSN: 0002-7863
- LANGHALS H ET AL: "A Two-Step Synthesis of Quaterrylenetetracarboxylic Bisimides-Novel NIR Fluorescent Dyes" TETRAHEDRON LETTERS, ELSEVIER, AMSTERDAM, vol. 36, no. 36, 4 septembre 1995 (1995-09-04), pages 6423-6424, XP004027248 ISSN: 0040-4039
- HOLTRUP F O ET AL: "TERRYLENIMIDES: NEW NIR FLUORESCENT DYES" CHEMISTRY - A EUROPEAN JOURNAL, WILEY - V C H VERLAG GMBH & CO. KGAA, WEINHEIM, DE, vol. 3, no. 2, 1 janvier 1997 (1997-01-01), pages 219-225, XP000931226 ISSN: 0947-6539
- QUANTE H ET AL: "QUATERRYLENEBIS(DICARBOXIMIDES)" ANGEWANDTE CHEMIE. INTERNATIONAL EDITION, WILEY VCH VERLAG, WEINHEIM, vol. 34, no. 12, 7 juillet 1995 (1995-07-07), pages 1323-1325, XP002002793 ISSN: 1433-7851
- ADACHI, MURATA, NAKAMURA: JOURNAL OF PHYSICAL CHEMISTRY, vol. 99, 1995, pages 14240-14246, XP002510276
- PSCHIRER N G ET AL: "Pentarylene- and Hexarylenebis(dicarboximide)s: Near- Infrared-Absorbing Polyaromatic Dyes" ANGEWANDTE CHEMIE. INTERNATIONAL EDITION, WILEY VCH VERLAG, WEINHEIM, vol. 45, 1 janvier 2006 (2006-01-01), pages 1401-1404, XP002474592 ISSN: 1433-7851
- DOKUNIKHIIN N S ET AL: "Transformations of polycyclic ketones. VI. Synthesis of peropyrene derivatives from 2H-phenalene-1,3-dione by action of hydrogen chloride" JOURNAL OF ORGANIC CHEMISTRY OF THE USSR, M A I K NAUKA - INTERPERIODICA, RU, vol. 10, 1 janvier 1974 (1974-01-01), pages 2423-2425, XP009123941 ISSN: 0022-3271

## Description

La présente invention concerne de nouvelles quinones et tétrones colorées, un procédé pour les synthétiser, des intermédiaires de synthèse nécessaires à cette synthèse, ainsi que différentes utilisations de ces quinones et tétrones notamment en tant que colorants et/ou transporteurs de charges.

L'obtention de cellules photovoltaïques organiques efficaces pose les défis suivants :
- la structuration des matériaux conducteurs de charges électriques et/ou d'excitons afin d'augmenter la longueur de diffusion des excitons et la mobilité des charges,
- l'absorption de la lumière sur toute la gamme du spectre solaire même dans l'infrarouge et en-deçà.

Les cristaux liquides ont déjà été proposés pour la fabrication des couches de semiconducteurs pour cellules photovoltaïques organiques (L. Schmidt-Mende, A. Fechtenkötter, K. Müllen, E. Moons, R. H. Friend et J.D. MacKenzie, Science, 2001, 293, 1119). En particulier des molécules ayant la forme de disques rigides avec des chaînes latérales flexibles se sont avérées former des phases mésogènes colonnaires potentiellement très intéressantes en tant que conducteurs de charges et/ou d'excitons pour des cellules photovoltaïques organiques.

Il existe toutefois actuellement peu de molécules organiques stables, capables à la fois de former des cristaux liquides colonnaires à température ambiante et présentant un coefficient d'absorption important dans l'infrarouge et en deçà, c'est-à-dire à des longueurs d'onde comprise entre 600 et 1000 nm.

La demande de brevet US 2006/0054987 décrit une tétrone ayant la structure suivante :

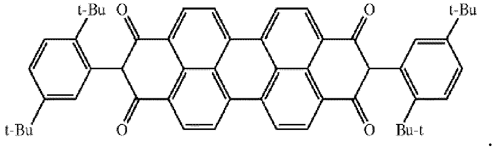

Le document Fluoreszenzfarbstoffe für Sonnenkollektoren, Rüdiger, Iden et al. Forschungsbericht - Bundesministerium für Forschung und Entwicklung, 1984, décrit une tétrone ayant la structure suivante :

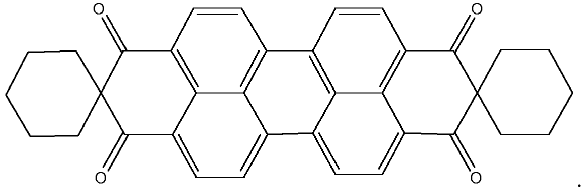

Le document Lee S. K et al., Electrochemistry, Spectroscopy and Electrogenerated Chemiluminescence of Perylene, Terrylene and Quaterrylene Diimides in Aprotic Solution, J. Am. Chem. Soc, 1999, 121, pp. 3513-3520, décrit des diimides ayant la structure suivante : 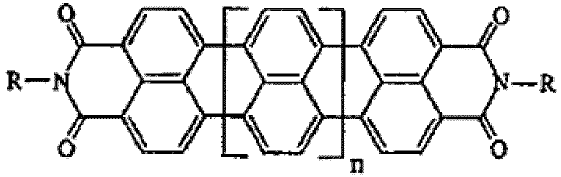 dans laquelle n = 0-2 et R réprésente un alkyl ou un aryl.

Dans ses recherches visant à mettre au point de nouveaux chromophores absorbant le rayonnement solaire de grande longueur d'onde et capables de former des mésophases, la Demanderesse a synthétisé de nouveaux homologues supérieurs des 2,7-dihydro-pyrène-1,3,6,8-tétrones. Ces quinones ont la structure générale suivante :

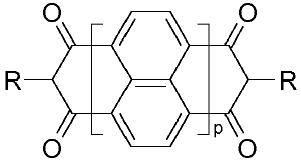

Les homologues supérieurs de ces quinones dans lesquels p > 2 n'ont jusqu'ici pas été décrits.

Ces quinones possèdent des fonctions 1,3-dione énolisables, ce qui confère à cette famille de composés une solubilité dans les bases telles que la pyridine. L'isomérie céto-énolique permet en outre l'introduction d'un ou de deux substituants par molécule, par exemple par estérification ou éthérification de la forme énol. Cette introduction de substituants est particulièrement intéressante pour les homologues supérieurs (p > 2) dont elle assure la solubilité dans les solvants organiques.

La Demanderesse a ainsi synthétisé une nouvelle famille de chromophores organiques et organosolubles, dichroïques et capables de former des phases mésogènes. Certaines de ces nouvelles molécules sont capables en particulier de former des cristaux liquides colonnaires et peuvent de ce fait être utilisées en tant que matériaux conducteurs de charges et/ou d'excitons dans des dispositifs photoélectriques. Le maximum d'absorption de ces nouveaux composés augmente avec la valeur de p et les homologues pour lesquels p est supérieur à 3 présentent une absorption dans l'infrarouge. La combinaison de différents représentants de cette famille ayant un système de doubles liaisons conjuguées plus ou moins important (avec p compris entre 1 et 6) permet ainsi de couvrir non seulement l'ensemble du spectre visible mais également la partie infrarouge du spectre solaire, ce qui est particulièrement intéressant dans la perspective d'une utilisation, en tant que chromophore et transporteur de charges et/ou d'excitons, dans des cellules photovoltaïques organiques.

La présente invention est telle que décrite dans les revendications.

La présente invention a ainsi pour objet de nouveaux composés de formules (Ia) et (Ib) 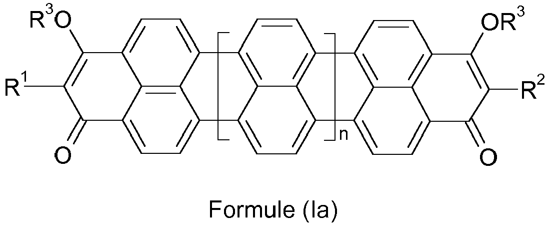 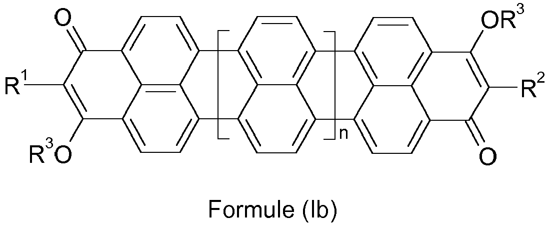 dans lesquelles
R¹ et R², identiques ou différents, représentent chacun indépendamment un groupe alkyle en C₅₋₂₅ ramifié,
chaque R³ représente indépendamment
   - un atome d'hydrogène,
   - un groupe alkyle en C₁₋₃₀, linéaire ou ramifié, dans lequel un ou plusieurs chaînons -CH₂-peuvent être remplacés par un atome d'oxygène, un atome de soufre, un groupe NH, N(alkyle en C₁₋₄) ou C=O, et qui est éventuellement substitué par un groupe aryle en C₆₋₁₀, hétéroaryle en C₄₋₁₀, cycloalkyle en C₃₋₂₀ ou hétérocycloalkyle en C₅₋₂₀, chacun de ces substituants pouvant à son tour porter un ou plusieurs substituants alkyle en C₁₋₂₄ ou alcoxyle en C₁₋₂₄, ou
   - un groupe aryle en C₆₋₁₀, hétéroaryle en C₄₋₁₀, (aryle en C₆₋₁₀)-carbonyle, (hétéroaryle en C₄₋₁₀)-carbonyle, chacun des ces groupes étant éventuellement substitué par un ou plusieurs substituants alkyle ou alcoxyle en C₁₋₂₄, aryle en C₆₋₁₀ ou hétéroaryle en C₄₋₁₀, cycloalkyle en C₃₋₂₀ ou hétérocycloalkyle en C₅₋₂₀,
n est un nombre entier entre 1 et 5 inclus, de préférence un nombre égal à 1 ou 2.

Lorsqu'un des groupes R³ ou les deux représentent un atome d'hydrogène, chacune des fonctions énol est en équilibre d'isomérisation avec la fonction cétone. Ainsi, par exemple pour le cas où les deux symboles R³ représentent chacun un atome d'hydrogène, les cinq formes isomères ci-après existent et sont toujours implicitement englobées lorsqu'une de ces formes est mentionnée ou représentée. C'est la raison pour laquelle le terme « quinones » utilisé dans la présente invention englobe à la fois les composés à deux groupes carbonyle et à quatre groupes carbonyle (tétrones)

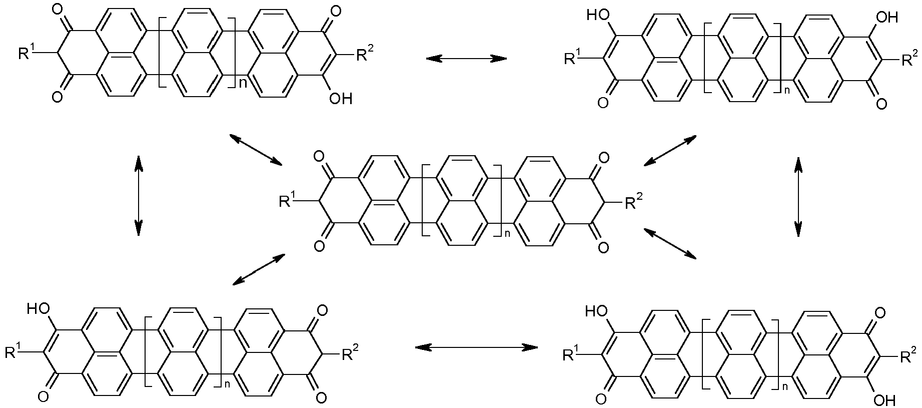

Dans les quinones de formule (Ia) et (Ib), R¹ et R² représentent chacun indépendamment un groupe alkyle en C₅ - C₂₅ ramifié, en particulier un groupe alkyle en C₈-C₁₈ ramifié. La ramification se situe de préférence en position α ou β par rapport au point de rattachement du groupe R¹ ou R² au noyau quinone. De telles ramifications proches du noyau rigide ont une influence positive sur la solubilité (S. Demmig, H. Langhals, Chem. Ber., 1988, 121, 255 ; H. Langhals, J. Büttner, P. Blanke, Synthesis 2005, 3, 364). On peut citer parmi ces résidus ramifiés en position bêta les groupes 2-éthylhexyle ou 2-hexyldécyle.

Bien que la présente invention couvre les quinones non-substituées sur les atomes d'oxygène (les deux R³ = H), les quinones mono-substituées (un seul R³ = H) et les quinones disubstituées (les deux R³ ≠ H) de formule (Ia) et (Ib), les dérivés disubstitués sont préférés parce qu'ils présentent une bonne solubilité dans les solvants organiques. Dans un mode de réalisation avantageux des nouveaux composés, chaque R³ représente indépendamment un groupe acyle, de préférence un groupe acyle de formule (-C=O)-R⁵ où R⁵ est un groupe alkyle en C₁₋₂₃.

Si la synthèse de la 2,9-dihydropéropyrène-1,3,8,10-tétrone (n = 0, R¹=R²=H) par dimérisation de la 2H-phénalène-1,3-dione dans du KOH fondu est connue (DE 283365 au nom de BASF), cette méthode s'avère toutefois inefficace pour des composés où R¹ et R² sont des substituants longs et ramifiés. La Demanderesse a découvert qu'il était possible d'augmenter considérablement le rendement de cette synthèse en remplaçant le KOH par du RbOH et encore mieux par du CsOH.

La présente divulgation décritégalement un procédé de synthèse de dihydropéropyrènetétrones (n = 0) de formules (Ia) et/ou (Ib) par dimérisation de phénalènediones substituées.

La première étape (étape (a)) de ce procédé consiste à faire réagir, deux phénalène-1,3-diones de formule 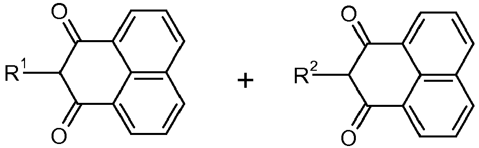 où R¹ et R² ont la signification et les préférences indiquées ci-dessus,
dans de l'hydroxyde de césium (CsOH) ou de l'hydroxyde de rubidium (RbOH) fondu, à une température supérieure ou égale à 200 °C, de préférence supérieure ou égale à 220 °C, de préférence pendant au moins une heure, en particulier pendant au moins 2 heures, de manière à obtenir un composé de formule II 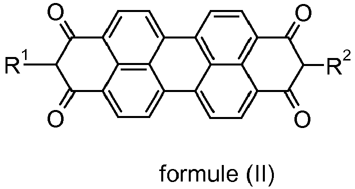 dans laquelle R¹ et R² ont les significations et préférences indiquées ci-dessus. Ce composé de formule (II), en équilibre avec les isomères énol correspondants, est un cas particulier des composés de formules (Ia) et (Ib) où les deux substituants R³ représentent un atome d'hydrogène.

Dans le procédé de synthèse décrit ci-dessus, on peut ensuite faire réagir ce composé de formule (II), dans une deuxième étape (b), avec au moins un équivalent d'un réactif de formule R³ - X où R³ représente :
- un groupe alkyle en C₁₋₃₀, linéaire ou ramifié, dans lequel un ou plusieurs chaînons -CH₂-peuvent être remplacés par un atome d'oxygène, un atome de soufre, un groupe NH, N(alkyle en C₁₋₄) ou C=O, et qui est éventuellement substitué par un groupe aryle en C₆₋₁₀, hétéroaryle en C₄₋₁₀, cycloalkyle en C₃₋₂₀ ou hétérocycloalkyle en C₅₋₂₀, chacun de ces substituants pouvant à son tour porter un ou plusieurs substituants alkyle en C₁₋₂₄ ou alcoxyle en C₁₋₂₄, ou
- un groupe aryle en C₆₋₁₀, hétéroaryle en C₄₋₁₀, (aryle en C₆₋₁₀)-carbonyle, (hétéroaryle en C₄₋₁₀)-carbonyle, chacun des ces groupes étant éventuellement substitué par un ou plusieurs substituants alkyle ou alcoxyle en C₁₋₂₄, aryle en C₆₋₁₀ ou hétéroaryle en C₄₋₁₀, cycloalkyle en C₃₋₂₀ ou hétérocycloalkyle en C₅₋₂₀,
et X représente un groupe partant approprié, choisi par exemple parmi les groupes halogéno, sulfonate et hydroxyle,
de manière à obtenir un composé de formule (Ia) et/ou (Ib) où n = 0 et au moins l'un des R³ ne représente pas un atome d'hydrogène.

La synthèse de quinones de rang supérieur (n = 1 à 5) peut se faire par cyclisation de précurseurs de formule (III) 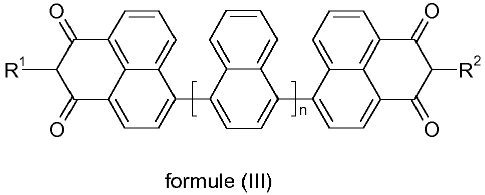 où n = 1 - 5 et R¹ et R² ont les significations indiquées ci-dessus, préalablement synthétisés à partir d'éléments terminaux portant les groupes R¹ et R² et d'éléments centraux formant l'enchaînement de noyaux naphtalènes. Ces précurseurs sont des composés nouveaux et constituent par conséquent un autre objet de la présente invention.

Dans cette réaction de cyclisation d'un composé de formule (III), le KOH s'est également avéré inefficace en tant que milieu réactionnel lorsque R¹ et R² sont de taille importante, mais la synthèse se déroule de manière satisfaisante lorsqu'on utilise, à sa place, de l'hydroxyde de rubidium ou de l'hydroxyde de césium.

La présente invention a par conséquent pour objet un procédé de synthèse de quinones de formule (Ia) ou (Ib), dans lequel on soumet, dans une première étape (a), un composé de formule (III) 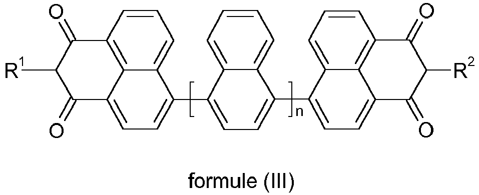 où R¹ et R² ont la signification et les préférences indiquées ci-dessus, et n = 1 - 5, de préférence n = 1 ou 2, à une réaction de cyclisation dans de l'hydroxyde de césium (CsOH) ou de l'hydroxyde de rubidium (RbOH), à une température supérieure ou égale 200 °C, de préférence supérieure ou égale à 220 °C, de préférence pendant au moins une heure, en particulier au moins deux heures, de manière à obtenir un composé de formule IV 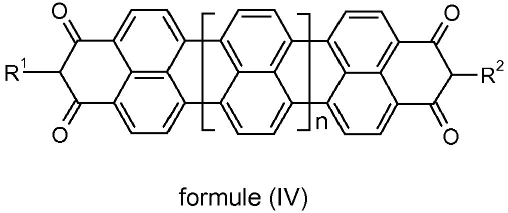 dans laquelle R¹ et R² ont les significations indiquées ci-dessus et n = 1 - 5.

Pour obtenir des quinones substituées, c'est-à-dire des quinones de formule (Ia) et/ou (Ib) où les symboles R³ ne représentent pas tous les deux un atome d'hydrogène, on fait réagir le composé de formule (IV) obtenu, dans une deuxième étape (b), avec au moins un équivalent d'un réactif de formule R³ - X où R³ représente :
- un groupe alkyle en C₁₋₃₀, linéaire ou ramifié, dans lequel un ou plusieurs chaînons -CH₂-peuvent être remplacés par un atome d'oxygène, un atome de soufre, un groupe NH, N(alkyle en C₁₋₄) ou C=O, et qui est éventuellement substitué par un groupe aryle en C₆₋₁₀, hétéroaryle en C₄₋₁₀, cycloalkyle en C₃₋₂₀ ou hétérocycloalkyle en C₅₋₂₀, chacun de ces substituants pouvant à son tour porter un ou plusieurs substituants alkyle en C₁₋₂₄ ou alcoxyle en C₁₋₂₄, ou
- un groupe aryle en C₆₋₁₀, hétéroaryle en C₄₋₁₀, (aryle en C₆₋₁₀)-carbonyle, (hétéroaryle en C₄₋₁₀)-carbonyle, chacun des ces groupes étant éventuellement substitué par un ou plusieurs substituants alkyle ou alcoxyle en C₁₋₂₄, aryle en C₆₋₁₀ ou hétéroaryle en C₄₋₁₀, cycloalkyle en C₃₋₂₀ ou hétérocycloalkyle en C₅₋₂₀,
et X représente un groupe partant approprié, choisi par exemple parmi les groupes halogéno, sulfonate et hydroxyle,
de manière à obtenir un composé de formule (Ia) et/ou (Ib) où n = 1 - 5 et au moins l'un des R³ ne représente pas un atome d'hydrogène.

Dans les deux procédés de synthèse indiqués ci-dessus, à savoir la synthèse de dihydropéropyrènetétrones (n = 0) par dimérisation de phénalènediones et la synthèse de quinones de rang supérieur (n = 1 - 5) par cyclisation d'un précurseur de formule (III), l'étape (a) est de préférence mise en oeuvre à une température comprise entre 240 °C et 300 °C, en particulier entre 260 °C et 280 °C, pendant une durée comprise entre 2 et 5 heures, de préférence entre 2,5 et 3,5 heures.

En tant que milieu réactionnel, on préfère tout particulièrement l'hydroxyde de césium, dans lequel les rendements réactionnels sont globalement meilleurs que dans l'hydroxyde de rubidium et qui permet, dans certains cas, de mettre en oeuvre la réaction à une température plus basse et avec une durée réactionnelle inférieure.

Comme indiqué ci-dessus, la synthèse du nouveau précurseur de formule (III) peut se faire par assemblage d'éléments terminaux (phénalènediones portant un groupe R (=R¹ et/ou R²)) et d'éléments centraux bifonctionnels dérivés du naphtalène selon le schéma réactionnel général suivant : 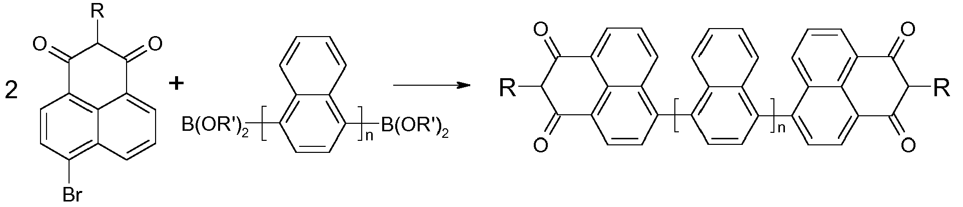 où n est compris entre 1 et 5, R = R¹ et/ou R² et chaque R' représente un atome d'hydrogène ou un groupe alkyle ou les deux R' d'un même groupe forment un résidu alkylène.

Bien que les phénalènediones bromées, représentées ci-dessus, aient une réactivité particulièrement satisfaisante dans cette réaction, l'utilisation des analogues chlorés peut également être envisagée.

Cette voie de synthèse présente de nombreux avantages :
- les types de réactions mis en jeu sont limités (principalement couplage de Suzuki et cyclisation)
- les éléments de départ sont soit des produits commerciaux soit facilement accessibles à partir de produits commerciaux,
- la symétrie de synthèse permet de réduire le nombre d'étapes grâce à des réactions simultanées,
- la stratégie est modulable. En fonction des éventuelles difficultés rencontrées, on peut en effet intervertir les fonctions -Br et -B(OR')₂ au niveau des réactifs de départ et le procédé de la présente invention englobe également cette autre variante. Le choix de l'atome de brome ou de chlore sur l'élément terminal et des groupes boronate sur l'élément central permet une plus grande convergence de la synthèse que la configuration inverse.

La phénalènedione bromée (élément terminal) peut être synthétisée selon la réaction suivante entre un malonate de dialkyle substitué en position 2 et la 6-bromo-2H-phénalène-1,3-dione, produit commercial : 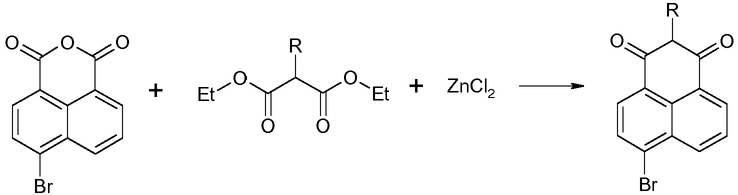 en suivant le protocole décrit dans G. Errera, Gazetta Chim. Ital. 1911, 1, page 190. Bien entendu, la phénalènedione non bromée peut être préparée de façon analogue par réaction entre ce même malonate de dialkyle et la 2H-phénalène-1,3-dione qui est également un produit commercial.

La 2H-phénalène-1,3-dione substituée en position 2 ainsi obtenue, qu'elle soit substituée ou non substituée en position 6, se trouve bien entendu dans une isomérie de céto-énolisation avec la 3-hydroxy-phénalèn-1-one et la 1-hydroxy-phénalèn-3-one substituée en position 2 correspondante. La mention d'un de ces composés englobe dans la présente demande toujours également les autres.

Les éléments centraux peuvent être préparés de la manière suivante :
Pour n = 1 : 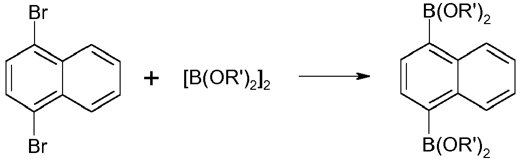
   Le 1,4-dibromonaphtalène est un produit commercial. La synthèse de l'acide naphtalène-1,4-diboronique (R' = H) est décrite par exemple dans Zang et al., J. Org. Chem., 2000, 65, pages 3952-3960 et Koch et al., Chem. Ber., 1991, 124, pages 2091 - 2100, celle du naphtalène-1,4-diboronate d'alkyle est décrite par exemple dans Ishiyama et al. J. Org. Chem. 1995, 60, pages 7508 - 7510.
Pour n = 2 : 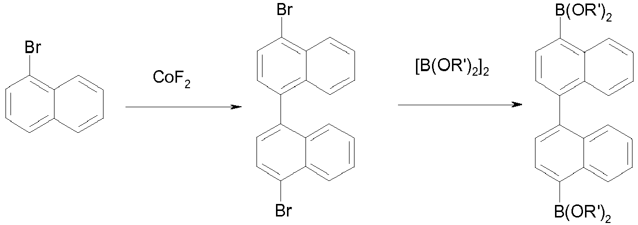
   Le 1-bromonaphtalène est un produit commercial. Sa dimérisation en présence de fluorure de cobalt est décrite par exemple dans McKillop et al., J. Am. Chem. Soc. 1980, 102, 6504 - 6512. La conversion du 4,4'-dibromo-1,1'-dinaphtyle en acide diboronique ou diboronate se fait par analogie avec la réaction indiquée ci-dessus pour n = 1.
Pour n = 3 : 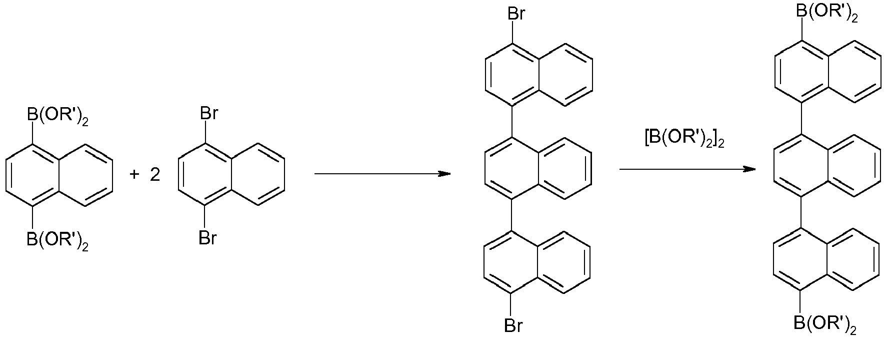
Pour n = 4 : 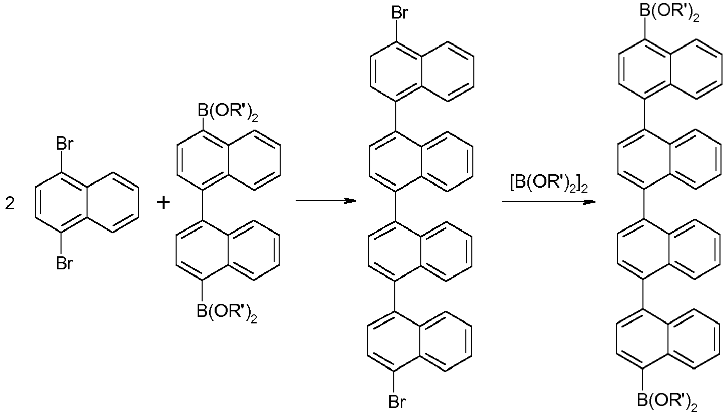
Pour n = 5 :

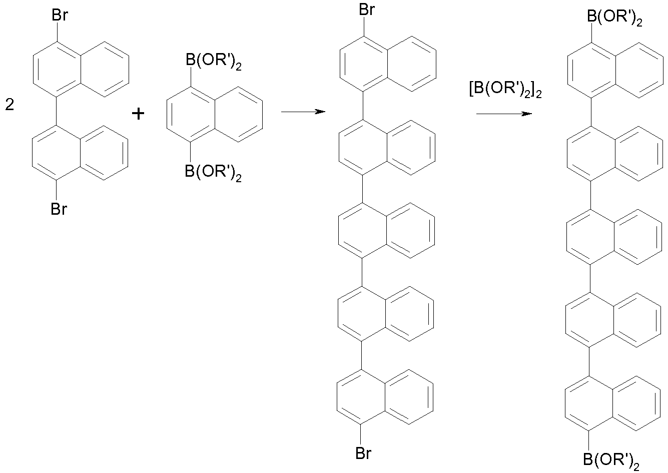

Ces deux derniers schémas réactionnels (n = 4 ou 5) sont une combinaison d'étapes réactionnelles décrites ci-dessus pour n = 1 - 3.

Enfin, une dernière variante de synthèse d'un composé de formule (Ia) ou (Ib) où n = 2 ou 4 comprend, dans un premier temps, le couplage d'une phénalènedione monobromée portant un groupe R (=R¹ et/ou R²) avec un dérivé monofonctionnel de naphtalène ou de 1,1-dinaphtyle respectivement selon le schéma réactionnel général suivant : 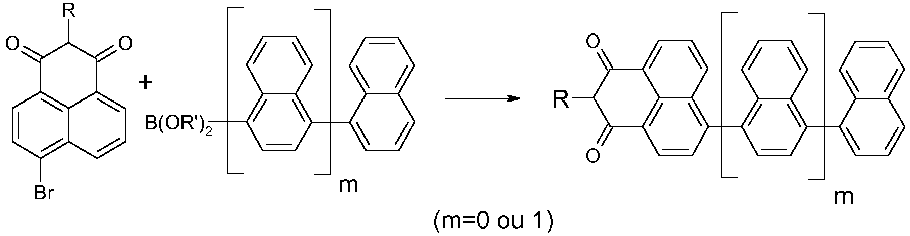 puis la dimérisation et la cyclisation du composé ainsi obtenu dans de l'hydroxyde de césium (CsOH) ou de l'hydroxyde de rubidium (RbOH) fondu, à une température supérieure ou égale à 200 °C, de préférence supérieure à 220 °C, de préférence pendant au moins une heure, en particulier pendant au moins 2 heures :

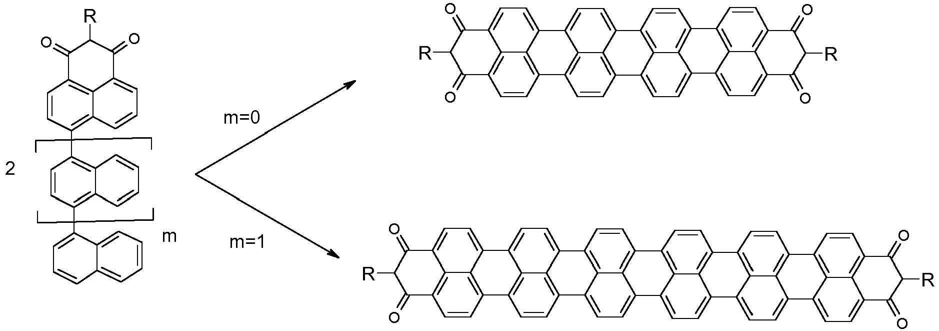

La présente invention a en outre pour objet l'utilisation des composés de formule (Ia) et (Ib) en tant que colorants, en particulier en tant que colorants dichroïques.

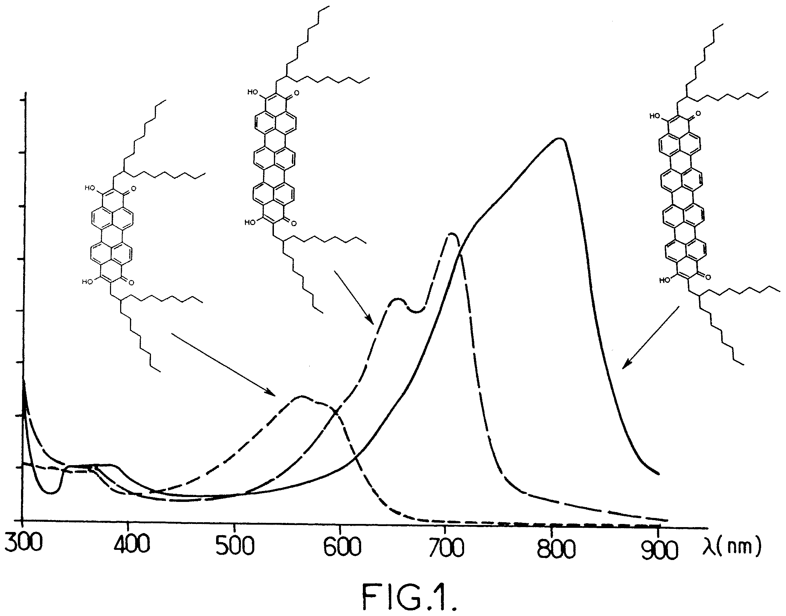

Ces composés présentent en effet une forte absorption dans le spectre visible et/ou dans l'infrarouge. La longueur d'onde correspondant au maximum d'absorption est d'autant plus importante que la valeur de n est élevée, c'est-à-dire que le système de doubles liaisons conjuguées est étendu. La figure 1 montre le spectre d'absorption, dans la pyridine, de trois composés qui diffèrent uniquement par la valeur de n allant de 0 à 2.

On peut constater que la combinaison de plusieurs composés selon l'invention permet ainsi d'obtenir des compositions ou matériaux absorbant dans le domaine des grandes longueurs d'ondes du spectre visible et dans l'infrarouge (600 nm et au-delà).

Leur bonne solubilité dans la plupart des solvants organiques permet d'utiliser les composés de formules (Ia) et (Ib) par exemple en tant que colorants dans des encres. Elle permet également le dépôt de couches minces de ces colorants à partir de solutions, bien que le dépôt de couches minces par sublimation ou évaporation sous vide puisse également être envisagé.

Les composés de formules (Ia) et (Ib) de la présente invention sont des colorants dichroïques et peuvent par conséquent être utilisés avantageusement dans des dispositifs où ils seront alignés de façon anisotrope dans l'espace, par exemple grâce à un support orienté, grâce à la présence d'une autre phase mésogène, suite à l'application d'un courant, ou simplement grâce à leur capacité intrinsèque à former une phase mésogène. On peut citer à titre d'exemples de tels dispositifs les dispositifs d'affichage à cristaux liquides ou les polariseurs.

Les composés de la présente invention peuvent former des mésophases. En particulier ceux de formules (Ia) et (Ib), où R³ ne représente pas un atome d'hydrogène, sont capables de former des structures de type cristaux liquides colonnaires. Cette propriété permet de les utiliser en tant que composants de cellules photovoltaïques, de diodes émettrices de lumière et de transistors à effet de champ où ils peuvent jouer le rôle de transporteurs de charges et/ou d'excitons, et, dans le cas des cellules photovoltaïques, de colorant absorbant la lumière visible et/ou infrarouge.

La présente invention a par conséquent également pour objet l'utilisation d'un composé de formules (Ia) et (Ib) en tant que transporteur de charges et/ou d'excitons.

### Exemples

### Synthèse de 2,9-di(2-éthylhexyl)-3,10-dihydroxy-péropyrène-1,8-quinone (composé de formule Ia/Ib non compris dans la présente invention avec n = 0, R³ = H et R¹ et R² = 2-éthylhexyle) par dimérisation de 2-(2-éthylhexyl)-3-hydroxyphénalèn-1-one

Un mélange d'hydroxyde de césium monohydraté (10,40 g, 61,93 mmoles) et de 2-(2-éthylhexyl)-3-hydroxyphénalèn-1-one (1,015 g, 3,29 mmoles) est chauffé à 280 °C pendant 3 heures dans un creuset métallique. Après refroidissement à température ambiante, le produit brut est dissous dans 300 mL d'eau et la solution violette obtenue est acidifiée par addition d'acide sulfurique dilué (15 mL dans 100 mL d'eau), ce qui donne un solide de couleur noire. On extrait le mélange avec 250 mL de butan-1-ol. On combine les phases organiques et on évapore le solvant sous pression réduite. La recristallisation du produit obtenu dans du méthanol donne 627 mg de 2,9-di(2-éthylhexyl)-3,10-dihydroxy-péropyrène-1,8-quinone (rendement 60 %)

### Synthèse de 2,9-di(2-hexyldécyl)-3,10-dihydroxy-péropyrène-1,8-quinone (composé de formule Ia/Ib non compris dans la présente invention avec n = 0, R³ = H et R¹ et R² = 2-hexyldécyle) par dimérisation de 2-(2-hexyldécyl)-3-hydroxy-phénalèn-1-one

On chauffe un mélange d'hydroxyde de césium monohydraté (9,00 g, 53,59 mmoles) et de 2-(2-hexyldécyl)-3-hydroxy-phénalèn-1-one (0,971 g, 2,31 mmoles) pendant 3 heures à 280 °C dans un creuset métallique.

Après refroidissement à température ambiante, on dissout le produit brut dans un mélange de 250 mL d'eau et de 250 mL d'acétone. On agite la solution obtenue pendant quelques minutes à température ambiante, puis on l'acidifie par addition d'acide sulfurique dilué (15 mL dans 100 mL d'eau). On extrait ensuite le mélange avec 250 mL de chloroforme. On combine les phases organiques et on évapore le solvant sous pression réduite. On purifie le produit obtenu par chromatographie sur colonne de gel de silice en éluant d'abord avec du chloroforme stabilisé avec de l'amylène, puis avec du chloroforme stabilisé avec 0,6 % d'EtOH, et pour finir avec un mélange chloroforme :acétate d'éthyle (gradient de 10/1 à 5/1). La recristallisation de l'huile ainsi obtenue dans du méthanol fournit 207 mg de 2,9-di(2-hexyldécyl)-3,10-dihydroxy-péropyrène-1,8-quinone (rendement 21 %).

### Synthèse de 2,11-di(2-hexyldécyl)-3,12-dihydroxy-téropyrène-1,10-quinone (composé de formule (Ia/Ib) où n = 1, R¹ et R² = 2-hexyldécyle, R³ = H) par cyclisation de 1,4-bis-(2-(2-hexyldécyl)-3-hydroxy-phénalèn-1-on-6-yl)naphtalène

On chauffe un mélange d'hydroxyde de césium monohydraté (10,32 g, 61,45 mmoles) et de 1,4-bis-(2-(2-hexyldécyl)-3-hydroxy-phénalèn-1-on-6-yl)naphtalène (1,019 g, 1,06 mmoles) pendant 3 heures à 280 °C dans un creuset métallique.

Après refroidissement à température ambiante, on dissout le produit brut dans un mélange de 200 mL d'eau et de 200 mL d'acétone. On agite la solution obtenue pendant quelques minutes à température ambiante, puis on l'acidifie par addition d'acide sulfurique dilué (15 mL dans 100 mL d'eau). On extrait ensuite le mélange avec 250 mL de chloroforme. On combine les phases organiques et on évapore le solvant sous pression réduite. On remet en suspension le produit obtenu pendant 40 minutes dans de l'éthanol à reflux puis on récupère par filtration 569 mg (rendement 65 %) de 2,11-di(2-hexyldécyl)-3,12-dihydroxy-téropyrène-1,10-quinone.

### Synthèse de 2,13-di(2-hexyldécyl)-3,14-dihydroxy-quatéropyrène-1,12-quinone (composé de formule (Ib/Ia) où n = 2, R³ = H et R¹ et R² = 2-hexyldécyle) par cyclisation de 4,4'-bis-(2-(2-hexyldécyl)-3-hydroxy-phénalèn-1-on-yl)-1,1'-binaphtyle

On chauffe un mélange d'hydroxyde de césium monohydraté (9,02 g, 53,71 mmoles) et de 4,4'-bis-(2-(2-hexyldécyl)-3-hydroxy-phénalèn-1-on-yl)-1,1'-binaphtyle (0,993 g, 0,91 mmole) pendant 3 heures à 280 °C dans un creuset métallique.

Après refroidissement à température ambiante, on dissout le brut réactionnel dans un mélange de 200 mL d'eau et de 200 mL d'acétone. On agite la solution obtenue pendant quelques minutes à température ambiante, puis on l'acidifie par addition d'acide sulfurique dilué (15 mL dans 100 mL d'eau). On extrait ensuite le mélange avec 250 mL d'acétate d'éthyle. On combine les phases organiques et on évapore le solvant sous pression réduite. On remet en suspension le produit obtenu pendant 40 minutes dans de l'éthanol à reflux puis on récupère par filtration 640 mg de 2,13-di(2-hexyldécyl)-3,14-dihydroxy-quatéropyrène-1,12-quinone (rendement 65 %).

### Estérification de 2,9-di(2-éthylhexyl)-3,10-dihydroxy-péropyrène-1,8-quinone (n = 0, R₁ et R₂ = 2-éthylehexyle, non compris dans la présente invention) avec du chlorure de 2-éthylhexanoyle (R³ = 2-étylhexanoyle) et séparation des isomères cis et trans

On ajoute lentement 4 mL (23,09 mmoles) de chlorure de 2-éthylhexanoyle à un mélange de 2,9-di(2-éthylhexyl)-3,10-dihydroxy-péropyrène-1,8-quinone (677,5 mg, 1,11 mmoles) et de pyridine (5 ml, 61,82 mmoles) dans 20 mL de chloroforme stabilisé à l'amylène. On agite le mélange violet obtenu à température ambiante et à l'abri de l'humidité. On dilue ensuite le produit brut dans 250 mL de chloroforme et on acidifie avec du HCl dilué (50 mL de HCl à 37 % dans 250 mL d'eau). On extrait ensuite le mélange avec 50 mL d'eau et 50 mL de chloroforme. On combine les phases organiques, on sèche sur sulfate de sodium anhydre, puis on évapore le solvant sous pression réduite. On purifie le liquide violet obtenu par chromatographie sur colonne de gel de silice en éluant d'abord avec du chloroforme stabilisé à l'amylène, puis avec du chloroforme stabilisé avec 0,6 % d'éthanol. On obtient ainsi deux solides colorés (le moins polaire est de couleur violette et le plus polaire de couleur bleue).

La recristallisation de ces solides dans du méthanol donne les deux isomères :
Isomère *trans* (composé de formule (Ib) : (2,9-di(2-éthylhexyl)-3,10-di(2-éthylhexanoyloxy)-péropyrène-1,8-quinone) : 326 mg de solide violet (0,38 mmole, rendement 34 %)
Isomère *cis* (composé de formule (Ia) : (2,9-di(2-éthylhexyl)-3,8-di(2-éthylhexanoyloxy)-péropyrène-1,10-quinone): 273 mg d'un solide bleu (0,32 mmole, rendement 29 %)

### Estérification de 2,9-di(2-hexyldécyl)-3,10-dihydroxy-péropyrène-1,8-quinone (n = 0, R¹ et R² = 2-éthylehexyle, non compris dans la présente invention) avec du chlorure de 2-éthylhexanoyle (R³ = 2-étylhexanoyle) et séparation des isomères cis et trans

On ajoute lentement 4 mL (23,09 mmoles) de chlorure de 2-éthylhexanoyle à un mélange de 2,9-di(2-hexyldécyl)-3,10-dihydroxy-péropyrène-1,8-quinone (336,4 mg, 0,40 mmole) et de pyridine (5 mL, 61,82 mmoles) dans 20 mL de chloroforme stabilisé à l'amylène. On agite le mélange violet obtenu à température ambiante et à l'abri de l'humidité. On dilue ensuite le produit brut dans 250 mL de chloroforme et on acidifie avec du HCl dilué (50 mL de HCl à 37 % dans 250 mL d'eau). On extrait ensuite le mélange avec 50 mL d'eau et 50 mL de chloroforme. On combine les phases organiques, on sèche sur sulfate de sodium anhydre, puis on évapore le solvant sous pression réduite. On purifie le liquide violet obtenu par chromatographie sur colonne de gel de silice en éluant d'abord avec du chloroforme stabilisé à l'amylène, puis avec du chloroforme stabilisé avec 0,6 % d'éthanol. On obtient ainsi deux solides colorés (le moins polaire est de couleur violette et le plus polaire de couleur bleue).

La recristallisation de ces solides dans du méthanol donne les deux isomères :
Isomère *trans* (composé de formule (Ib) (2,9-di(2-hexyldécyl)-3,10-di(2-éthylhexanoyloxy)-péropyrène-1,8-quinone) : 23,5 mg de solide violet (0,02 mmole, rendement 5 %)
Isomère *cis* (composé de formule (Ia) (2,9-di(2-hexyldécyl)-3,8-di(2-éthylhexanoyloxy)-péropyrène-1,10-quinone): 72,7 mg d'un solide bleu (0,07 mmole, rendement 17 %)

### Synthèse des intermédiaires de synthèse 2-(2-éthylhexyl)-3-hydroxy-phénalèn-1-one (R¹ = R² = 2-éthylhexyle)

On effectue la synthèse de cet intermédiaire selon G. Errera, Gazetta Chim. Ital. 1911, 1, 190.

On dissout 13,6 g (200 mmoles) d'éthylate de sodium dans 100 mL d'éthanol anhydre chauffé à reflux. On ajoute, toujours à reflux, 32 g (200 mmoles) de malonate de diéthyle, puis 30 minutes plus tard 38,6 g (200 mmoles) de 2-éthyl-1-bromohexane. Après quelques minutes, du bromure de sodium commence à précipiter. Après 12 heures de chauffage à reflux, on ajoute le mélange réactionnel dans 500 mL d'acide chlorhydrique à 5 % et on extrait le mélange obtenu avec 500 mL d'acétate d'éthyle. On sèche la phase organique sur du sulfate de sodium et l'on élimine le solvant par évaporation. On élimine le malonate d'éthyle n'ayant pas réagi par distillation à 170 °C sous pression réduite (trompe à eau) et on utilise le résidu, du (2-éthylhexyl)malonate de diéthyle brut, sans purification supplémentaire. Dans un flacon de 250 mL, on ajoute à ce (2-éthylhexyl)malonate de diéthyle 19,8 g (100 mmoles) d'anhydride 1,8-naphtalénique et 30 g (220 mmoles) de dichlorure de zinc sous agitation vigoureuse. On maintient l'agitation pendant 5 heures en chauffant à 200 °C. A ce moment, le dégagement de gaz a presque cessé. On transfère le fluide visqueux marron dans un flacon de 1 litre, puis on ajoute 400 mL d'acétate d'éthyle et une solution de 60 g de d'hydroxyde de potassium dans 400 mL d'eau. Après agitation vigoureuse pendant 15 minutes, on élimine les sels de zinc précipités en filtrant le mélange biphasique sur un grand filtre de verre. On lave la phase aqueuse avec de l'acétate d'éthyle. On lave les phases organiques réunies avec de l'acide chlorhydrique dilué et on évapore le solvant. On dissout le résidu dans du chloroforme et on filtre sur une colonne de gel de silice large et courte afin de séparer les impuretés polaires de couleur sombre. Après évaporation du solvant, on recristallise le produit dans de l'heptane. Rendement : 9,6 g (31 mmoles, rendement 31 %) d'un solide jaune poisseux.

### 2-(2-hexyldécyl)-3-hydroxy-phénalèn-1-one (R = 2-hexyldécyle)

On répète le protocole précédent en utilisant 61 g (200 mmoles) de 2-hexyl-1-bromodécane à la place du 2-éthyl-1-bromohexane. On recristallise le produit obtenu dans trois litres de pentane (3 jours au congélateur). Une quantité inférieure de pentane donne moins de précipité. On obtient 9,8 g (23 mmoles, rendement 23 %) d'un solide jaune poisseux.

### 6-bromo-2-(2-hexyldécyl)-3-hydroxy-phénalèn-1-one

On répète le protocole précédant en utilisant 27,7 g (100 mmoles) d'anhydride 4-bromo-1,8-naphtalénique au lieu de l'anhydride 1,8-naphtalénique. Rendement : 12,5 g (25 mmoles, 25 %).

### Diester cyclique d'acide naphtalène-1,4-diboronique et de pinacol

On dissout un mélange de 5,03 g (17,59 mmoles) de 1,4-dibromonaphtalène, 17,70 g (69,70 mmoles) de bis(pinacolato)dibore et 16,99 g (173,12 mmoles) d'acétate de potassium dans 50 mL de 1,4-dioxane sous atmosphère d'argon à température ambiante. On ajoute 1 g (1,22 mmole) de chlorure de 1,1'-bis(diphénylphosphino)ferrocène Pd^{II} (PdCl₂-dppf) en solution dans 20 mL de 1,4-dioxane. On agite le mélange obtenu pendant 16 heures à 90 °C. Après refroidissement, le mélange réactionnel est lavé avec de l'eau et du chloroforme. On poursuit l'extraction de la phase aqueuse avec du chloroforme. Les phases organiques sont combinées et séchées sur sulfate de sodium anhydre, puis on élimine le solvant par évaporation. Le produit brut est ensuite purifié par chromatographie colonne sur gel de silice avec élution par un mélange de cyclohexane/dichlorométhane (60/40). On obtient ainsi 3,09 g de diester cyclique d'acide naphtalène-1,4-diboronique et de pinacol (rendement 46 %).

### Diester cyclique d'acide 1,1'-binaphtyl-4,4'-diboronique et de pinacol

On agite un mélange de 7,21 g (17,49 mmoles) de 4,4'-dibromo-1,1'-binaphtyle, de 17,70 g (69,70 mmoles) de bis(pinacolato)dibore et de 17,04 g (173,63 mmoles) d'acétate de potassium dans 50 mL de 1,4-dioxane pendant quelques minutes à température ambiante et sous atmosphère d'argon jusqu'à dissolution. On ajoute 1 g (1,22 mmole) de chlorure de 1,1'-bis(diphénylphosphino)ferrocène Pd^{II} (PdCl₂-dppf) en solution dans 35 mL de 1,4-dioxane. On maintient l'agitation du mélange pendant 18 heures à 90 °C. Après refroidissement le mélange réactionnel est lavé avec de l'eau et du chloroforme. On poursuit l'extraction de la phase aqueuse avec du chloroforme. Les phases organiques sont combinées et séchées sur sulfate de sodium anhydre, puis on élimine le solvant par évaporation. Le produit brut est ensuite purifié par chromatographie colonne sur gel de silice en éluant successivement avec les mélanges suivants de cyclohexane/dichlorométhane : 3/1, 3/2, 2/1, puis avec du chloroforme pur. On obtient ainsi 5,76 g d'un mélange contenant 75 % de diester cyclique d'acide 1,1'-binaphtyl-4,4'-diboronique et de pinacol, et 25 % de pinacol.

### 1,4-bis-(2-(2-hexyldécyl)-3-hydroxy-phénalèn-1-on-6-yl)-naphtalène

On agite un mélange de 5,41 g (10,83 mmoles) de 6-bromo-2-(2-hexyldécyl)-3-hydroxy-phénalène-1-one et de 0,45 g (0,42 mmoles) de tétrakis-(triphénylphosphino)-Pd⁽⁰⁾ dans 20 mL de 1,2-diméthoxyéthane (DME) pendant 10 minutes à température ambiante et sous atmosphère d'argon.

On ajoute ensuite une solution de 1,84 g (4,84 mmoles) de diester cyclique d'acide naphtalène-1,4-diboronique et de pinacol dans 20 ml de 1,2-diméthoxyéthane (DME) et on agite le mélange pendant quelques minutes à température ambiante et sous atmosphère d'argon. Enfin, on ajoute une solution aqueuse de 10,27 g (96,90 mmoles) de carbonate de potassium dans 40 mL d'eau.

On chauffe le mélange obtenu pendant 17 heures à reflux (100 °C) sous agitation et sous un léger flux d'argon. Après refroidissement, on lave le brut réactionnel avec 200 mL d'eau et 200 mL de chloroforme, on le verse dans un bécher de 3 litres et l'on acidifie avec de l'acide chlorhydrique à 37 %.

On extrait ensuite la phase aqueuse trois fois avec 50 mL de chloroforme. On combine les phases organiques et on sèche sur sulfate de sodium anhydre, puis l'on élimine les solvants par évaporation sous vide. On purifie ensuite le produit brut par chromatographie sur colonne de gel de silice en éluant d'abord avec du dichlorométhane, puis avec un gradient dichlorométhane/acétate d'éthyle (90/10 à 30/20). On recueille ainsi, après évaporation du solvant, un solide de couleur orange que l'on recristallise dans de l'heptane, après filtration à chaud (reflux). La recristallisation fournit 3,35 g (rendement 72 %) de 1,4-bis-(2-(2-hexyldécyl)-3-hydroxy-phénalèn-1-on-6-yl)-naphtalène.

### 4,4'-bis-(2-(2-hexyldécyl)-3-hydroxy-phénalèn-1-on-6-yl)-1,1'-dinaphtyle

On agite un mélange de 4,22 g (8,45 mmoles) de 6-bromo-2-(2-hexyldécyl)-3-hydroxy-phénalène-1-one et de 0,36 g (0,31 mmoles) de tétrakis-(triphénylphosphino)-Pd⁽⁰⁾ dans 20 mL de 1,2-diméthoxyéthane (DME) pendant 10 minutes à température ambiante et sous atmosphère d'argon.

On ajoute ensuite une solution de 2,01 g du mélange contenant 75 % de diester cyclique d'acide 1,1'-binaphtyl-4,4'-diboronique et de pinacol et 25 % de pinacol dans 20 mL de 1,2-diméthoxyéthane (DME) et on agite le mélange pendant quelques minutes à température ambiante sous atmosphère d'argon.

Pour finir, on ajoute une solution aqueuse de 8,16 g de carbonate de sodium (76,99 mmoles) dans 40 mL d'eau.

On chauffe le mélange obtenu pendant 17 heures à reflux (100 °C) sous agitation et sous un léger flux d'argon. Après refroidissement, on lave le brut réactionnel avec 200 mL d'eau et 200 mL de chloroforme, on le verse dans un bécher de 3 litres et l'on acidifie avec de l'acide chlorhydrique à 37 %.

On extrait ensuite la phase aqueuse avec 100 mL de chloroforme. On combine les phases organiques et on sèche sur du sulfate de sodium anhydre, puis on élimine le solvant par évaporation sous vide. On purifie ensuite le produit brut par chromatographie sur colonne de gel de silice en éluant d'abord avec du chloroforme stabilisé avec de l'amylène, puis avec un mélange chloroforme/acétate d'éthyle (90/10), ce qui donne un solide de couleur jaune que l'on recristallise dans un mélange d'acétate d'éthyle/heptane (1/3). On obtient ainsi 2,41 g (rendement 73 %) de 4,4'-bis-(2-(2-hexyldécyl)-3-hydroxy-phénalèn-1-on-6-yl)-1,1'-dinaphtyle.

## Revendications

1. Quinone de formule (Ia) ou (Ib) 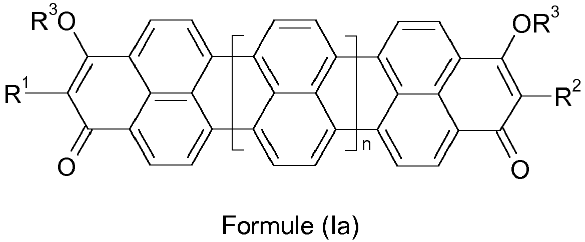 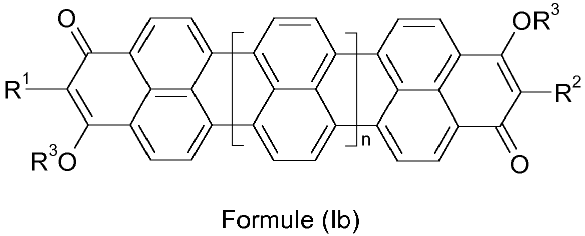 dans lesquelles
R¹ et R², identiques ou différents, représentent chacun indépendamment
- un groupe alkyle en C₅ - C₂₅ ramifié,
chaque R³ représente indépendamment
- un atome d'hydrogène,
- un groupe alkyle en C₁₋₃₀, linéaire ou ramifié, dans lequel un ou plusieurs chaînons -CH₂-peuvent être remplacés par un atome d'oxygène, un atome de soufre, un groupe NH, N(alkyle en C₁₋₂₄) ou C=O, ou
- un groupe aryle en C₆₋₁₀, hétéroaryle en C₄₋₁₀, (aryle en C₆₋₁₀)-carbonyle, (hétéroaryle en C₄₋₁₀)-carbonyle,
n est un nombre entier entre 1 et 5 inclus.

2. Quinone de formule (Ia) ou (Ib) selon la revendication 1, **caractérisée par le fait que** n est un nombre entier égal à 1 ou 2.

3. Quinone de formule (Ia) ou (Ib) selon la revendication 1, **caractérisée par le fait que** R¹ et R² représentent chacun indépendamment un groupe alkyle ramifié en C₈ - C₁₈.

4. Quinone de formule (Ia) ou (Ib) selon la revendication 3, **caractérisée par le fait que** R¹ et R² représentent chacun indépendamment un groupe alkyle en C₈ - C₁₈ ramifié en position α ou β par rapport à leur point de rattachement.

5. Quinone de formule (Ia) ou (Ib) selon l'une des revendications 1 ou 3, **caractérisée par le fait que** R¹ et R² représentent chacun indépendamment un groupe 2-éthylhexyle ou 2-hexyldécyle.

6. Quinone de formule (Ia) ou (Ib) selon l'une quelconque des revendications précédentes, **caractérisée par le fait que** chaque R³ représente un groupe acyle.

7. Quinone de formule (Ia) ou (Ib) selon la revendication 6, **caractérisée par le fait que** chaque R³ représente un groupe acyle de formule (-C=O)-R⁵ où R⁵ est un groupe alkyle en C₁₋₂₃.

8. Procédé de préparation d'une quinone de formule (Ia) ou (Ib) telle que définie à l'une quelconque des revendications 1 à 7, **caractérisé par le fait que** l'on soumet, dans une première étape (a), un composé de formule (III) 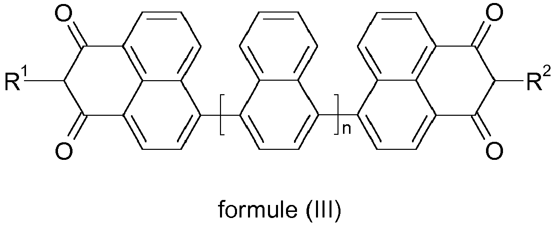 où R¹ et R² ont la signification indiquée dans l'une quelconque des revendications 1 à 7 et n = 1 - 5, à une réaction de cyclisation dans de l'hydroxyde de césium (CsOH) ou de l'hydroxyde de rubidium (RbOH), à une température supérieure ou égale à 200 °C, de manière à obtenir un composé de formule IV 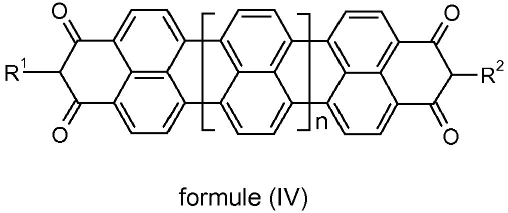 dans laquelle R¹ et R² ont les significations indiquées ci-dessus et n = 1 - 5.

9. Procédé selon la revendication 8, **caractérisé par le fait que** l'on fait réagir le composé de formule (IV), dans une deuxième étape (b), avec au moins un équivalent d'un réactif de formule R³ - X où R³ a la signification telle indiquée dans la revendication 1, à l'exception d'un atome d'hydrogène, et X représente un groupe partant approprié, de manière à obtenir un composé de formule (Ia) et/ou (Ib) où n = 1 - 5 et au moins l'un des R³ ne représente pas un atome d'hydrogène.

10. Procédé selon la revendication 8, **caractérisé par le fait que** l'étape (a) est mise en oeuvre à une température comprise entre 240 °C et 300 °C, pendant une durée comprise entre 2 et 5 heures.

11. Procédé selon la revendication 10, **caractérisé par le fait que** l'étape (a) est mise en oeuvre à une température comprise 260 °C et 280 °C, pendant une durée comprise entre 2,5 et 3,5 heures.

12. Procédé selon l'une quelconque des revendications 8 à 11, **caractérisé par le fait que** l'étape (a) est mise en oeuvre dans l'hydroxyde de césium.

13. Composé de formule 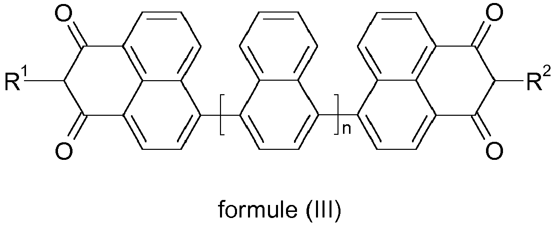 où R¹ et R² ont la signification indiquées dans l'une quelconque des revendications 1 - 7 et n = 1 - 5.

14. Utilisation d'une quinone selon l'une quelconque des revendications 1 à 7, en tant que colorant.

15. Utilisation d'un composé selon l'une quelconque des revendications 1 à 7 en tant que transporteur de charges et/ou d'excitons.

## Patentansprüche

1. Chinon der Formel (Ia) oder (Ib) 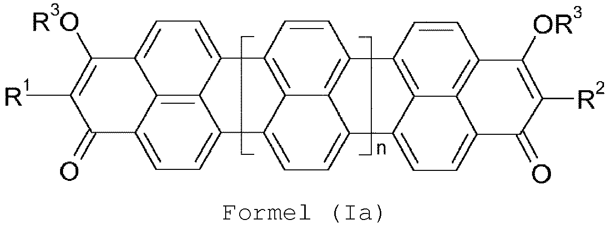 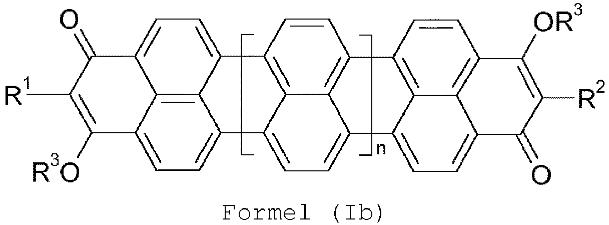 in denen
R¹ und R² gleich oder verschieden sind und jeweils unabhängig für
- eine verzweigte C₅-C₂₅-Alkylgruppe stehen,
R³ jeweils unabhängig für
- ein Wasserstoffatom,
- eine lineare oder verzweigte C₁₋₃₀-Alkylgruppe, in der ein oder mehrere -CH₂-Glieder durch ein Sauerstoffatom, ein Schwefelatom oder eine NH-, N(C₁₋₂₄-Alkyl)- oder C=O-Gruppe ersetzt sein können, oder
- eine C₆₋₁₀-Aryl-, C₄₋₁₀-Heteroaryl-, (C₆₋₁₀-Aryl)-carbonyl- oder (C₄₋₁₀-Heteroaryl) carbonylgruppe steht,
n für eine ganze Zahl zwischen 1 und 5 inklusive steht.

2. Chinon der Formel (Ia) oder (Ib) nach Anspruch 1, **dadurch gekennzeichnet, dass** n für eine ganze Zahl mit einem Wert von 1 oder 2 steht.

3. Chinon der Formel (Ia) oder (Ib) nach Anspruch 1, **dadurch gekennzeichnet, dass** R¹ und R² jeweils unabhängig für eine verzweigte C₈-C₁₈-Alkylgruppe stehen.

4. Chinon der Formel (Ia) oder (Ib) nach Anspruch 3, **dadurch gekennzeichnet, dass** R¹ und R² jeweils unabhängig für eine C₈-C₁₈-Alkylgruppe, die in α- oder β-Position bezüglich ihres Anbindungspunkts verzweigt ist, stehen.

5. Chinon der Formel (Ia) oder (Ib) nach einem der Ansprüche 1 oder 3, **dadurch gekennzeichnet, dass** R¹ und R² jeweils unabhängig für eine 2-Ethylhexyl- oder 2-Hexyldecylgruppe stehen.

6. Chinon der Formel (Ia) oder (Ib) nach einem der vorhergehenden Ansprüche, **dadurch gekennzeichnet, dass** R³ jeweils für eine Acylgruppe steht.

7. Chinon der Formel (Ia) oder (Ib) nach Anspruch 6, **dadurch gekennzeichnet, dass** R³ jeweils für eine Acylgruppe der Formel (-C=O)-R⁵ steht, wobei R⁵ für eine C₁₋₂₃-Alkylgruppe steht.

8. Verfahren zur Herstellung eines Chinons der Formel (Ia) oder (Ib) gemäß einem der Ansprüche 1 bis 7, **dadurch gekennzeichnet, dass** man in einem ersten Schritt (a) eine Verbindung der Formel (III) 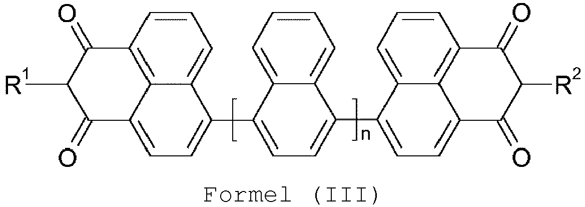 wobei R¹ und R² die in einem der Ansprüche 1 bis 7 angegebene Bedeutung haben und n = 1-5, einer Cyclisierungsreaktion in Caesiumhydroxid (CsOH) oder Rubidiumhydroxid (RbOH) bei einer Temperatur größer oder gleich 200 °C unterwirft, wobei man eine Verbindung der Formel IV 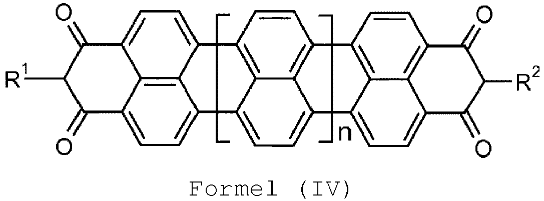 in der R¹ und R² die oben angegebenen Bedeutungen haben und n = 1-5, erhält.

9. Verfahren nach Anspruch 8, **dadurch gekennzeichnet, dass** man die Verbindung der Formel (IV) in einem zweiten Schritt (b) mit mindestens einem Äquivalent eines Reagenz der Formel R³-X, wobei R³ die in Anspruch 1 angegebene Bedeutung mit Ausnahme eines Wasserstoffatoms hat und X für eine geeignete Abgangsgruppe steht, umsetzt, wobei man eine Verbindung der Formel (Ia) und/oder (Ib), wobei m = 1-5 und mindestens eine der Variablen R³ nicht für ein Wasserstoffatom steht, erhält.

10. Verfahren nach Anspruch 8, **dadurch gekennzeichnet, dass** der Schritt (a) bei einer Temperatur zwischen 240 °C und 300 °C über einen Zeitraum zwischen 2 und 5 Stunden durchgeführt wird.

11. Verfahren nach Anspruch 10, **dadurch gekennzeichnet, dass** der Schritt (a) bei einer Temperatur zwischen 260 °C und 280 °C über einen Zeitraum zwischen 2,5 und 3,5 Stunden durchgeführt wird.

12. Verfahren nach einem der Ansprüche 8 bis 11, **dadurch gekennzeichnet, dass** der Schritt (a) in Caesiumhydroxid durchgeführt wird.

13. Verbindung der Formel 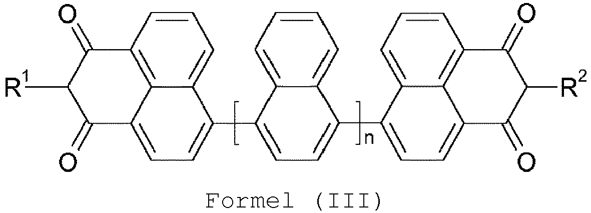 wobei R¹ und R² die in einem der Ansprüche 1-7 angegebene Bedeutung haben und n = 1-5.

14. Verwendung eines Chinons nach einem der Ansprüche 1 bis 7 als Farbmittel.

15. Verwendung einer Verbindung nach einem der Ansprüche 1 bis 7 als Ladungs- und/oder Exziton-Transporter.

## Claims

1. Quinone of formula (Ia) or (Ib) 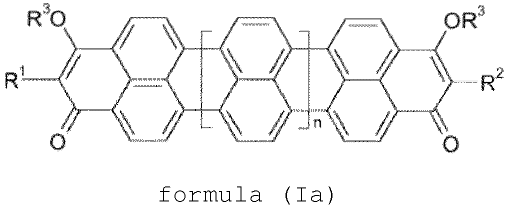 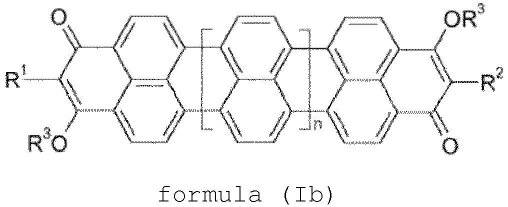 in which
R¹ and R², which may be identical or different, each independently represent
- a branched C₅-C₂₅ alkyl group,
each R³ independently represents
- a hydrogen atom,
- a linear or branched C₁-C₃₀ alkyl group, in which one or more -CH₂- chain members can be replaced with an oxygen atom, a sulfur atom, or an NH, N (C₁-C₂₄ alkyl) or C=O group, or
- a C₆-C₁₀ aryl, C₄-C₁₀ heteroaryl, (C₆-C₁₀ aryl) carbonyl, or (C₄-C₁₀ heteroaryl)carbonyl group,
n is an integer between 1 and 5 inclusive.

2. Quinone of formula (Ia) or (Ib) according to Claim 1, **characterized in that** n is an integer equal to 1 or 2.

3. Quinone of formula (Ia) or (Ib) according to Claim 1, **characterized in that** R¹ and R² each independently represent a branched C₈-C₁₈ alkyl group.

4. Quinone of formula (Ia) or (Ib) according to Claim 3, **characterized in that** R¹ and R² each independently represent a C₈-C₁₈ alkyl group which is branched in the α- or β-position relative to their point of attachment.

5. Quinone of formula (Ia) or (Ib) according to either of Claims 1 and 3, **characterized in that** R¹ and R² each independently represent a 2-ethylhexyl or 2-hexyldecyl group.

6. Quinone of formula (Ia) or (Ib) according to any one of the preceding claims, **characterized in that** each R³ represents an acyl group.

7. Quinone of formula (Ia) or (Ib) according to Claim 6, **characterized in that** each R³ represents an acyl group of formula (-C=O)-R⁵ wherein R⁵ is a C₁-C₂₃ alkyl group.

8. Process for preparing a quinone of formula (Ia) or (Ib) as defined in any one of Claims 1 to 7, **characterized in that**, in a first step (a), a compound of formula (III) 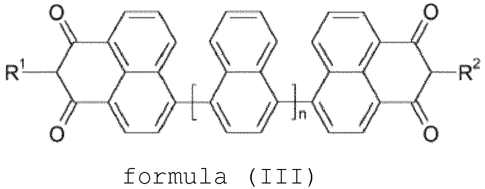 wherein R¹ and R² have the meaning indicated in any one of Claims 1 to 7 and n = 1-5, is subjected to a cyclization reaction in caesium hydroxide (CsOH) or rubidium hydroxide (RbOH), at a temperature greater than or equal to 200°C, so as to obtain a compound of formula IV 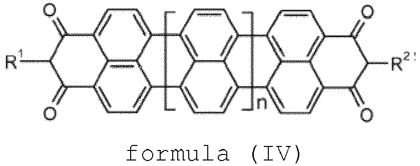 in which R¹ and R² have the meanings indicated above and n = 1-5.

9. Process according to Claim 8, **characterized in that**, in a second step (b), the compound of formula (IV) is reacted with at least one equivalent of a reagent of formula R³-X, wherein R³ has the meaning as indicated in Claim 1, with the exception of a hydrogen atom, and X represents an appropriate leaving group, so as to obtain a compound of formula (Ia) and/or (Ib) wherein n = 1-5 and at least one of the R³ groups does not represent a hydrogen atom.

10. Process according to Claim 8, **characterized in that** step (a) is carried out at a temperature between 240°C and 300°C, for a period of between 2 and 5 hours.

11. Process according to Claim 10, **characterized in that** step (a) is carried out at a temperature between 260°C and 280°C, for a period of between 2.5 and 3.5 hours.

12. Process according to any one of Claims 8 to 11, **characterized in that** step (a) is carried out in caesium hydroxide.

13. Compound of formula 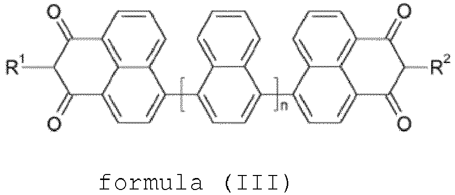 wherein R¹ and R² have the meanings indicated in any one of Claims 1-7 and n = 1-5.

14. Use of a quinone according to any one of Claims 1 to 7, as a dye.

15. Use of a compound according to any one of Claims 1 to 7, as a charge and/or exciton transporter.
